Europäisches Patentamt

European Patent Office

Office européen des brevets

(11) Numéro de publication: **0 307 871 B1**

# (12) FASCICULE DE BREVET EUROPEEN

(45) Date de publication de fascicule du brevet: **23.12.92**   (51) Int. Cl.⁵: **A61F  13/00**

(21) Numéro de dépôt: **88114961.1**

(22) Date de dépôt: **13.09.88**

(54) **Bande élastique en matériau textile, procédé de fabrication d'une telle bande, et son utilisation en tant que bande de pansement.**

(30) Priorité: **16.09.87 FR 8712830**

(43) Date de publication de la demande:
**22.03.89 Bulletin  89/12**

(45) Mention de la délivrance du brevet:
**23.12.92 Bulletin  92/52**

(84) Etats contractants désignés:
**AT BE CH DE ES FR GB GR IT LI LU NL SE**

(56) Documents cités:
**FR-A- 2 035 319**
**FR-A- 2 207 685**
**US-A- 3 125 093**

(73) Titulaire: **PEAUDOUCE**
**59, Rue de la Vignette**
**F-59126 Linselles(FR)**

(72) Inventeur: **Koczab, Jean-Pierre**
**253 Domaine de la Vigne**
**F-59910 Bondues(FR)**

(74) Mandataire: **Casalonga, Axel et al**
**BUREAU D.A. CASALONGA - JOSSE Moras-sistrasse 8**
**W-8000 München 5(DE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention se rapporte à une bande élastique en matériau textile, à un procédé de fabrication d'une telle bande, et à son utilisation dans le domaine du pansement, en tant que bande de contention, de compression et/ou de maintien de pansements.

En tant que matériaux textiles élastiques en bande, on connaît principalement des matériaux constitués de fils susceptibles d'être allongés élastiquement, et des matériaux faisant appel à l'élasticité en flexion des fils constitutifs, ces derniers étant alors liés, par exemple par tricotage, de manière à former des boucles élastiquement déformables.

Dans le premier cas, il n'est possible d'utiliser que des fils de matières (élastomères) bien déterminées, généralement d'un prix de revient relativement élevé, et dans le second cas, il est nécessaire de lier les fils par des procédés (tricotage) qui réduisent considérablement la vitesse de fabrication, et augmentent ainsi le prix de revient du produit.

Enfin, le document FR-A-2 207 685 propose un bandage constitué par une bande de non-tissé de fibres cellulosiques entremêlées et maintenues assemblées grâce à des vrilles, coudes, torsades et boucles induits dans les fibres, cette bande présentant une série répétitive d'ondulations transversales obtenue par une action de crêpage ou gaufrage sur la bande de non-tissé, de telle manière que les ondulations présentent une forte densité des fibres dans la zone des crêtes et des creux des ondulations et une faible densité des fibres dans les flancs des ondulations. Toutefois, suivant ce document, l'action de crêpage ou de gaufrage est une action mécanique entraînant un réarrangement des fibres maintenues assemblées, à l'état entremêlé, uniquement par frottement, c'est-à-dire par engagement mécanique. Dans ces conditions, la capacité d'allongement élastique de la bande ainsi rétractée par ce crêpage ne peut être que faible. Par ailleurs, ce crêpage effectué de manière à produire des ondulations s'étendant sur toute la largeur de la bande donne à cette dernière partout le même aspect et le même toucher.

La présente invention vise une bande élastique pouvant être réalisée à partir de matériaux textiles bon marché, à une cadence de fabrication élevée, cette bande ayant une capacité d'allongement élastique élevée et un aspect textile ainsi qu' un toucher doux.

L'invention vise également un procédé de fabrication simple, rapide et peu coûteux pour la réalisation d'une telle bande élastique.

Enfin, l'invention vise l'utilisation d'une telle bande élastique en tant que bande de pansement jetable après usage.

Telle que revendiquée, la bande élastique conforme à l'invention en matériau textile est constituée par une bande de non-tissé présentant suivant la direction longitudinale une succession d'ondulations transversales produisant une réduction de la longueur de la bande. Suivant l'invention, le non-tissé est formé en partie au moins de fibres fusibles en matière thermoplastique et comporte des ondulations permanentes obtenues par crêpage à chaud du non-tissé de telle manière que la bande présente dans le sens de la largeur des bandes longitudinales fortement crêpées séparées par des bandes longitudinales faiblement crêpées.

Une telle ondulation obtenue par un crêpage permanent à chaud permet d'utiliser un non-tissé constitué, en partie au moins, de n'importe quelles fibres thermoplastiques. En effet, ces fibres, telles qu'elles sont utilisées couramment dans les non-tissés servant par exemple de feuille intérieure perméable aux liquides sur des couches-culottes et des articles d'hygiène analogues, peuvent être soumises à un crêpage à chaud qui susbsiste après refroidissement et qui procure aux fibres une élasticité due non pas à l'élasticité en allongement, mais à l'élasticité en flexion des fibres. Sur des fibres liées dans un non-tissé, l'ondulation obtenue par un crêpage de l'ensemble du non-tissé procure à ce dernier une capacité d'allongement élastique qui est d'autant plus importante que les ondulations sont plus hautes et plus serrées. La prévision, sur la bande de non-tissé, dans le sens de sa largeur, de zones alternées fortement crêpées et faiblement crêpées donne à la bande un aspect de côtes longitudinales rappelant l'aspect d'un textile, notamment d'un tricot, ainsi qu'un toucher doux et "volumineux", les zones faiblement crêpées étant gonflées par rapport aux zones fortement crêpées.

Pour la fabrication d'une bande de non-tissé élastique conforme à l'invention, il est prévu, suivant l'invention, de procéder au crêpage en faisant passer le non-tissé, porté à une température sensiblement égale à la température de ramollissement des fibres fusibles constitutives du non-tissé, entre la surface périphérique, munie de sillons périphériques espacés, d'un cylindre entraîné en rotation et une lame en appui élastique sur la surface périphérique du cylindre en faisant un angle aigu avec cette surface dans le sens de rotation du cylindre.

Ce procédé de crêpage qui est connu en soi pour le crêpage de tissu permet, selon la pression d'appui de la lame sur la surface périphérique du cylindre, de donner au non-tissé un effet de crêpage serré conduisant à un taux de rétraction de 50% et même davantage, permettant ensuite un allongement élastique de 100% et davantage.

Du fait de la présence des sillons periphériques espacés du cylindre, le non-tissé ne subit, sous l'action de la lame en appui élastique sur la surface périphérique, un crêpage que dans les zones en contact avec les parties du cylindre situées entre les sillons.

La bande de non-tissé élastique conforme à l'invention peut être utilisée avantageusement en tant que bande élastique de contention, de compression ou de fixation de pansements, la bande pouvant être jetée après un usage unique, grâce à son prix de revient réduit.

Pour faciliter l'utilisation de la bande, il est avantageux que cette dernière présente à son extrémité correspondant à l'extrémité postérieure lors de la pose, un pavé d'adhésif permettant de fixer cette extrémité sans aucune difficulté par simple pression sur la spire précédente de la bande.

Afin d'empêcher ce pavé d'adhésif de coller sur la bande avant l'utilisation de cette dernière, le pavé d'adhésif peut être recouvert d'une feuille de protection facilement amovible, par exemple d'une feuille traitée à l'aide d'un agent anti-adhésif telle qu'une feuille de papier siliconé.

Suivant un autre mode de réalisation, la bande élastique conforme à l'invention peut être fabriquée et conditionnée sous la forme d'une bande de grande longueur présentant par intervalle des lignes transversales de perforations et des pavés d'adhésif. Cela permet, sans aucun outil, de détacher d'une bande de grande longueur des bandes individuelles en fonction des besoins, par déchirure à l'endroit des lignes transversales de perforations, chacune de ces bandes individuelles étant munie d'un pavé d'adhésif pour la fixation de l'extrémité de la bande en fin de pose de cette dernière.

En se référant aux dessins schématique annexé, on va décrire ci-après plus en détail un mode de réalisation illustratif et non limitatif de l'objet de l'invention; sur les dessins :

la figure 1 est une vue en plan d'une partie d'une bande élastique conforme à l'invention;

la figure 2 est une coupe à plus grande échelle suivant II-II de la figure 1;

la figure 3 représente une bande élastique conforme à l'invention posée autour d'un membre, avant la fixation par adhésif de l'extrémité postérieure de la bande;

la figure 4 représente très schématiquement un mode de fabrication de la bande conforme à l'invention;

la figure 5 est une coupe suivant V-V de la figure 4.

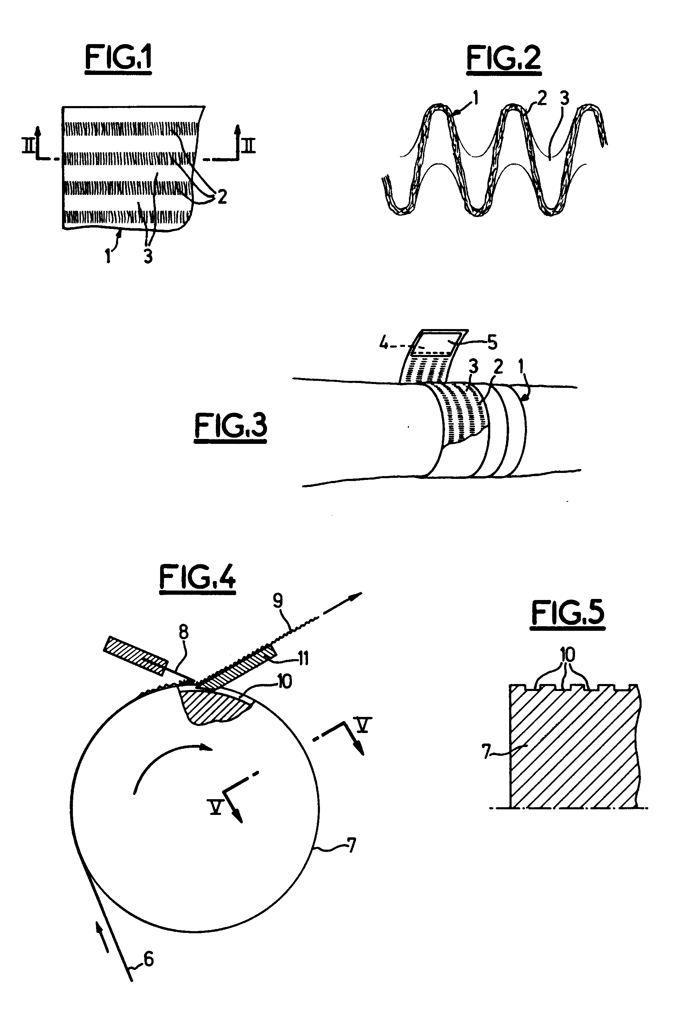

Selon la figure 1, une bande 1 d'un non-tissé contenant des fibres de matière thermoplastique est ondulée dans le sens longitudinal, c'est-à-dire présente des ondulations qui produisent une diminution de la longueur de la bande, ces ondulations ne s'étendant pas de façon continue sur toute la largeur de la bande 1, mais se présentant sous la forme de bandes longitudinales 2 fortement crêpées séparées par des bandes longitudinales 3 faiblement crêpées.

Ces ondulations sont dues à un crêpage permanent à chaud des fibres fusibles dans le non tissé 1.

La figure 2 qui est une coupe suivant II-II de la figure 1 à l'endroit d'une bande 2 fortement crêpée permet de reconnaître que dans les bandes 3 faiblement crêpée, le non-tissé 1 est également ondulé mais de façon plus lâche et présente un plus grand volume (plus grande épaisseur) que dans les bandes 2 fortement crêpées.

Le non-tissé peut être formé, en partie ou en totalité, de fibres de matières thermoplastiques quelconques, par exemple polyamide, polyester, polypropylène. De préférence, il contient en majorité des fibres de matières thermoplastiques. Le non-tissé peut également contenir des mélanges de fibres de plusieurs matières plastiques différentes.

La hauteur des ondulations résultant du crêpage et le pas des ondulations peuvent être choisis en fonction du taux d'allongement élastique recherché et de la force de rappel élastique que doit présenter le non tissé. Cette force de rappel élastique est d'autant plus élevée que les ondulations sont moins hautes et sont plus serrées.

Les largeurs des bandes 2 et fortement crêpées des bandes 3 faiblement crêpées peuvent également être choisies selon l'effet recherché (aspect textile, douceur, etc.).

Une application particulièrement intéressante d'une bande de non-tissé élastique conforme à l'invention consiste en l'utilisation de cette bande en tant que bande de contention, de compression et/ou de fixation de pansements. La bande conforme à l'invention assure, dans cette application, les mêmes fonctions que la bande élastique connue sous la dénomination de bande VELPEAU, mais en raison de son prix de revient nettement plus faible, la bande conforme à l'invention peut constituer une bande à usage unique, c'est-à-dire une bande à jeter après usage.

Selon la figure 3, une bande ondulée 1 conforme à l'invention utilisée en tant que bande de contention, de compression et/ou de maintien de pansements comporte, à son extrémité constituant l'extrémité postérieure de la bande après enroulement de cette dernière autour d'un membre, un pavé d'adhésif 4 recouvert d'une feuille de protection 5 qu'il suffit de détacher pour permettre la fixation de l'extrémité postérieure de la bande 1 sur la spire précédente de l'enroulement. Cela permet d'éviter l'utilisation des moyens supplémentaires nécessaires jusqu'à présent, par exemple des épingles de sûreté, des morceaux de ruban adhésif rapportés, etc.

Selon les figures 4 et 5, la bande élastique 1 conforme à l'invention peut être produite à partir d'une bande non-tissé 6 que l'on fait passer sur un rouleau 7 sur la surface périphérique de laquelle une lame 8 prend élastiquement appui, la lame faisant un angle aigu avec la surface périphérique du cylindre 7 dans le sens de rotation de ce dernier. Le cylindre 7 étant chauffé jusqu'à une température proche du point de ramollissement des fibres de matière thermoplastique contenues dans la bande de non-tissé 6, cette dernière subit, lors de son passage en dessous de la lame 8, un crêpage qui est fixé dans le non-tissé en raison du chauffage de ce dernier au contact du cylindre 7 chauffé. De ce fait, le non-tissé 9 quittant le cylindre 7 derrière la lame 8 présente une ondulation permanente.

On reconnaît sur les figures 4 et 5 que la surface périphérique du cylindre 7 comporte des gorges ou sillons espacés 10, de sorte que la lame 8 appuie sur la surface périphérique du cylindre 7 uniquement entre les sillons 10 et provoque donc un crêpage du non-tissé 6 essentiellement à ces endroits. De ce fait, la bande 9 de non-tissé quittant le cylindre 7 présente, comme le non-tissé 1 de la figure 1, des bandes fortement crêpées (2) séparées par des bandes faiblement crêpées (3).

Une racle 11 disposée derrière la lame 8 et présentant des dents engagées dans les sillons 10 de la surface latérale du cylindre 7 favorise le dégagement du non-tissé 9 ondulé du cylindre 7, en vue de son refroidissement avant enroulement.

Au lieu de chauffer le non-tissé 10 uniquement sur le cylindre 7 de crêpage, il serait également possible de le préchauffer, par exemple par passage sur un cylindre préalable ou par passage dans un four à rayons infrarouges.

Il serait également possible de crêper le non-tissé à froid et de le soumettre ensuite à une thermofixation, par exemple en le faisant passer dans une étuve de vaporisation avant de la refroidir.

A titre d'exemple, on a réalisé sur une machine du type MICREX selon la figure 4, avec un cylindre 7 chauffé, un non-tissé crêpé avec un taux de rétraction de 45%, à partir d'un non-tissé de 35 g/m$^2$ formé à 100% de fibres de polyamide 6. Ce non-tissé crêpé présente des côtes longitudinales (bandes 2 fortement crêpées d'une largeur de 2 mm séparée par des bandes 3 faiblement crêpées de 2 mm de large). Les fibres sont liées dans le non-tissé, avant crêpage, par des points de gaufrage à chaud à une densité d'environ 50 points par cm$^2$. Le même effet de crêpage peut être obtenu sur des non-tissés dans lesquels les fibres sont liées par d'autres procédés bien connus en soi.

**Revendications**

1. Bande élastique en matériau textile, constituée par une bande de non-tissé présentant suivant la direction longitudinale une succession d'ondulations transversales produisant une réduction de la longueur de la bande, caractérisée par le fait que le non-tissé est formé en partie au moins de fibres fusibles en matière thermoplastique et comporte des ondulations permanentes obtenues par crêpage à chaud du non-tissé de telle manière que la bande (1) présente dans le sens de la largeur des bandes longitudinales fortement crêpées (2) séparées par des bandes longitudinales faiblement crêpées (3).

2. Bande suivant la revendication 1, caractérisée par le fait qu'elle présente un crêpage correspondant à un taux de rétraction de 50% ou plus, permettant un allongement élastique de 100% ou plus de la bande.

3. Utilisation d'une bande de non-tissé suivant la revendication 1 ou 2 en tant que bande élastique de contention, de compression et/ou de fixation de pansements.

4. Bande suivant la revendication 3, caractérisé par le fait qu'elle présente un pavé d'adhésif (4) à l'une de ses extrémités.

5. Bande suivant la revendication 4, caractérisée par le fait que le pavé d'adhésif (4) est recouvert d'une feuille de protection (5).

6. Bande suivant la revendication 4 ou 5, caractérisée par le fait qu'elle est constituée par une bande de grande longueur présentant par intervalles des lignes transversales de perforations et des pavés d'adhésif.

7. Procédé de fabrication d'une bande élastique suivant l'une quelconque des revendications précédentes, caractérisée par le fait qu'on crêpe la bande de non-tissé en faisant passer cette dernière, portée à une température sensiblement égale au point de ramollissement des fibres fusibles du non-tissé, entre la surface périphérique, munie de sillons périphériques espacés, d'un cylindre entraîné en rotation et une lame en appui élastique sur la surface périphérique du cylindre en faisant un angle aigu avec la surface périphérique dans le sens de rotation du cylindre.

**Claims**

1. An elastic strip of textile material, comprising a strip of non-woven material having in the longitudinal direction a series of transverse undulations bringing about a reduction in the length of the strip, characterised in that the non-woven material is formed at least partly from fusible fibres of thermoplastic material and comprises permanent undulations obtained by hot crimping of the non-woven material in such a way that the strip (1) has, widthwise, heavily crimped longitudinal strips (2) which are separated by lightly crimped longitudinal strips (3).

2. A strip according to claim 1, characterised in that it is provided with crimping which corresponds to a contraction rate of 50 % or more, allowing elastic extension of 100 % or more of the strip.

3. Use of a strip of non-woven material according to claim 1 or 2 as a resilient strip for retention, compression and/or fastening dressings.

4. A strip according to claim 3, characterised in that it has an adhesive patch (4) at one of its ends.

5. A strip according to claim 4, characterised in that the adhesive patch (4) is covered with a protective sheet (5).

6. A strip according to claim 4 or 5, characterised in that it comprises a strip of great length provided at intervals with transverse lines of perforations and adhesive patches.

7. A method of producing an elastic strip according to any one of the preceding claims, characterised in that the strip of non-woven material is crimped by causing said strip, which has been brought to a temperature which is substantially equal to the softening point of the fusible fibres of the non-woven material, to pass between the peripheral surface of a cylinder, which is provided with peripheral spaced apart grooves and is driven in rotating motion, and a blade applied elastically on the peripheral surface of the cylinder and forming an acute angle with the peripheral surface in the direction of the rotation of the cylinder.

## Patentansprüche

1. Elastisches Band aus textilem Material, das aus einem Band aus Vliesstoff zusammengesetzt ist und in Längsrichtung eine Abfolge transversaler Kräuselungen aufweist, was zu einer Herabsetzung der Länge des Bandes führt,

dadurch **gekennzeichnet, daß**
der Vliesstoff zumindest teilweise aus schmelzbaren Fasern aus thermoplastischem Material gebildet ist und dauerhafte Kräuselungen aufweist, die durch ein in der Wärme durchgeführtes Kreppen des Vliesstoffes erhalten werden, und zwar auf eine solche Weise, daß das Band (1) in Richtung der Länge stark gekräuselte longitudinale Bandbereiche (2) aufweist, die von schwach gekräuselten longitudinalen Bandbereichen (3) abgesetzt sind.

2. Band gemäß Anspruch 1,
dadurch **gekennzeichnet, daß**
es eine Kräuselung entsprechend einem Kontraktionsfaktor von 50% oder mehr aufweist, was eine elastische Dehnbarkeit von 100% oder mehr des Bandes ermöglicht.

3. Verwendung eines Bandes aus Vliesstoff gemäß Anspruch 1 oder 2, als elastischer Stütz-, Druck- und/oder Fixierungsverband.

4. Band gemäß Anspruch 3,
dadurch **gekennzeichnet, daß**
es ein Haftpflaster (4) an einem seiner äußeren Enden aufweist.

5. Band gemäß Anspruch 4,
dadurch **gekennzeichnet, daß**
das Haftpflaster (4) durch ein Schutzblatt (5) abgedeckt ist.

6. Band gemäß Anspruch 4 oder 5,
dadurch **gekennzeichnet, daß**
es aus einem Band größerer Länge zusammengesetzt ist, das in Abständen voneinander transversale Perforationslinien und Haftpflasterbereiche aufweist.

7. Verfahren zur Herstellung eines elastischen Bandes gemäß jedem der vorhergehenden Ansprüche,
dadurch **gekennzeichnet, daß**
man das Band aus Vliesstoff kreppt, indem man das letztere, das bei einer Temperatur gehalten wird, die dem Erveichungspunkt der schmelzbaren Fasern des Vliesstoffes spürbar nahekommt, zwischen der mit in Abständen angeordneten äußeren Rillen versehenen Außenoberfläche eines in Rotation versetzten Zylinders und einer elastischen Druckplatte über der Außenfläche des Zylinders hindurchlaufen laßt, wobei ein spitzer Winkel mit der Aussenfläche in Drehrichtung des Zylinders gebildet wird.

## FIG.1

## FIG.2

## FIG.3

## FIG.4

## FIG.5